# EUROPEAN PATENT APPLICATION

(11) **EP 0 683 397 A1**
(43) Date of publication of application: **22.11.1995**
(21) Application number: 94905240.1
(22) Date of filing: 01.02.1994
(51) Int. Cl.: G01N 33/544

(54) **REAGENT FOR IMMUNOAGGLUTINATION AND IMMUNOANALYTICAL METHOD**

(30) Priority: 03.02.1993 JP 16167/93
(71) Applicant: NISSUI PHARMACEUTICAL CO., LTD., Toshima-ku, Tokyo 170 (JP)
(72) Inventor: UENO, Takahisa, Nissui Pharmaceutical Co. Ltd., Yuuki-shi, Ibaraki 307 (JP); UMEDA, Mamoru, Nissui Pharmaceutical Co. Ltd., Yuuki-shi Ibaraki 307 (JP); SHIBATA, Hideaki Nissui Pharmaceutical Co. Ltd., Yuuki-shi Ibaraki 307 (JP)
(74) Representative: Wächtershäuser, Günter, Prof. Dr.
(86) International application number: PCT/JP94/00136
(87) International publication number: WO 94/18567

(57) **Abstract**

A reagent for immunoagglutination to be used for determining the quantity of an antibody or antigen with a high sensitivity by causing the agglutination due to the antigen-antibody reaction of a liposomal agglutination reagent comprising an antibody or antigen immobilized onto the surface of a liposome as the support through covalent bonds and determining the quantity of the agglutination with the short wavelength of a light by which a turbidity change caused by the agglutination can be intensely observed. The liposome encloses therein a water-soluble high-molecular compound or a gelled compound, and the enclosed compound serves to increase the turbidity change caused by the agglutination caused by the antigen-antibody reaction.

## Description

### BACKGROUND OF THE INVENTION

### Field of the Invention

The present invention relates to an immunoagglutination reagent. More specifically, the present invention relates to an immunoagglutinationan reagent capable of assaying a wide concentration range of a substance present in a sample at a high sensitivity by a simple procedure or by using an automatic analyzer, and the present invention also relates to an immunoanalytical method using the same.

### Background Art

Immunoanalytical method using antigen-antibody reaction is extremely important for clinical diagnosis of endocrine diseases and the like. A variety of immunoanalytical methods have been known conventionally.

Among them, it has been known an immunoanalytical method comprising reacting an antibody or an antigenic substance in a sample with an immunoagglutination reagent comprising a carrier bound with an antigenic substance or an antibody, measuring the agglutination level caused by the antigen-antibody reaction under naked eye or as the turbidity with a spectrophotometer, comparing the results with a standard curve preliminary prepared, thereby assaying the antibody or the antigenic substance. Synthetic latex particles such as polystyrene have been used primarily as the carrier of the reagent immunoagglutination for such immunoanalytical method.

Because the surface of the reagent immunoagglutination using such conventional synthetic latex particles as the carrier is hydrophobic, agglutination via hydrophobic binding readily occurs in an aqueous solution of pH 7 to 7.5, suitable for antigen-antibody reaction. Thus, such reagent tends to induce non-specific agglutination. Therefore, the assay precision is likely to be unstable. Thus, patent applications (for example, Japanese Patent Publication No. Hei 3-142360, Japanese Patent Publication No. Hei 3-76425, etc.) have been submitted of the technique for adding additives to reagents immunoagglutination using synthetic latex particles as the carrier so as to suppress such non-specific agglutination.

Because an antigen or an antibody is immobilized via physical adsorption onto synthetic latex particles, the antigen or the antibody is dissociated under a long-term storage from the synthetic latex particles of such latex agglutination reagent immobilizing the antigen or the antibody, causing the decrease in assay sensitivity.

More recently, such latex agglutination reagents have been applied to general purpose biochemical automatic analysers, to assay an antigen or an antibody in a sample. However, it is a problem that an immunoagglutination reagent using the synthetic latex particles as a carrier adheres to the reaction cells of an automatic analyzer to give adverse effects on the assay precision.

So as to overcome these problems, the present inventors have developed a liposome agglutination reagent using as the carrier liposome covalently coated with an antigen or an antibody. Then, the inventors have submitted the application of the liposome agglutination reagent (Japanese Patent Application No. Hei 4-164783). Because an antigen or an antibody is covalently bound onto the surface of liposome in the liposome agglutination reagent, the antigen or the antibody is not dissociated from the liposomes even after a long-term storage. Because the surface of liposome of itself is hydrophilic, agglutination reaction via hydrophobic binding hardly occurs. Therefore, non-specific agglutination hardly occurs, advantageously.

The reagent has also an advantage in that the reaction cells of an automatic analyzer should never be contaminated, which advantage has never been found in conventional latex agglutination reagents.

Because latex agglutination reagents of themselves have higher turbidities, namely, because the blank of the reagents is high, such reagents should be used for assay with a spectrophotometer in a region of long wave length with less change in turbidity. Hence, the enhancement of the sensitivity thereof has been very hard. Alternatively, due to the lower refractive index, the liposome agglutination reagent characteristically can be used for assay in a region of short wave length, namely at about 340 nm, where larger turbidity change can be observed.

However, liposomes have a property with a small difference in refractive index from that of water. Additionally, liposomes are prepared in an aqueous solution and therefore, an aqueous solution is entrapped in the liposomes. Hence, almost no difference in refractive index is observed between the aqueous solution entrapped in the liposome and water as the external aqueous phase. Further, liposomes of themselves has a lower refractive index. Although the liposome agglutination reagent has advantages such that the turbidity thereof is not increased even if liposomes are agglutinated and the reagent can be assayed therefore at a short wave length of 340 nm, the liposome agglutination reagent has problems such that the assay sensitivity of the reagent is low even in case of using a spectrophotometer or an automatic analyzer or under naked eye.

Thus, overcoming such problems involved in the agglutination of the liposome agglutination reagent covalently coated with an antigen or an antibody onto a carrier liposome, the objective of the present invention is to provide an immunoagglutination reagent capable of highly sensitive assaying in a range of wave length where the change in turbidity via agglutination can be observed distinctively, namely at a short wave length.

Another objective of the present invention is to provide a high sesitive immunoagglutination reagent by using liposomes with an increased refractive index.

A further objective of the present invention is to provide an immunoanalytical method having a high sensitivity, even when a sample has a lower concentration.

### DISCLOSURE OF THE INVENTION

So as to overcome the problems described above, the present invention provides an immunoagglutination reagent to assay an agglutination level caused by an antigen-antibody reaction, wherein liposome is used as the carrier of the immunoagglutination reagent, an antibody or an antigen being immobilized onto the surface of the liposomes, and the liposomes entrapping a water-soluble polymer compound or a gelled compound.

In accordance with the present invention, the liposomes may be made of any conventional liposome material containing phospholipid and cholesterol as the principal components thereof. The particle size of liposome may be 50 nm to 5 µm, more preferably 100 to 600 nm, and most preferably 100 to 200 nm. In the range, the reagent is very stable because liposomes hardly precipitate under storage, and are preferably used for measuring the turbidity with a spectrophotometer or by a slide agglutination method or the like.

The water-soluble polymer compound or the gelled compound entrapped in the liposomes of the immunoagglutination reagent in accordance with the present invention may be made of a polymer or a copolymer such as acrylamide, gly cosylmethacrylate and the like; cellulose or the derivatives thereof; a methacrylate polymer; a polymer or a copolymer of acrylic acid or an acrylate ester; a polymer of N-vinylpyrollidone; and the like. These water-soluble polymer compounds or polymers should be crosslinked preliminarily via a crosslinking agent.

The immunoagglutination reagent in accordance with the present invention is produced by immobilizing an antibody or an antigen, for example, via covalent bonding onto the surface of liposome as described below. In accordance with the present invention, however, the immunoagglutination reagent comprises liposomes entrapping therein a water-soluble polymer compound or a gelled compound, with no specific limitation to the bonding form between an antibody or an antigen and the surface of liposomes. For example, the periodate process, the Passive process and the like, conventionally used, may be also applicable for the bonding between an antibody or an antigen and liposomes.

Description will follow firstly about the preparation of liposomes from phospholipid, cholesterol, and phospholipid introduced with a crosslinking agent, by the Vortexing process or a process by means of glass beads.

As the crosslinking agent to be used for the process, use may be made of, for example, N-hydroxysuccinimidyl 3-(2-pyridyldithio)propionate (SPDP), N-succinimidyl 4-(p-maleimide phenyl)butyrate (SMPB), N-succinimidyl 4-(p-maleimide phenyl) acetate (SMPA), N-succinimidyl 4-(p-maleimide phenyl)propionate (SMPP), N-(γ-maleimide butyryloxy)succinimide (GMBS) and N-(ε -maleimide caproyloxy)succinimide (EMCS) and the like.

The solvents contained in a mixture of phospholipid, cholesterol and phospholipid modified with a crosslinking agent are distilled off for drying under aspiration. Subsequently, adding an aqueous solution dissolving a polymerizable monomer into a flask with a thin film formed on the wall surface thereof, adding furthermore glass beads if necessary, and shaking the flask after sealing, a liposome suspension is prepared. Passing thereafter the suspension through a polycarbonate film of a given pore size, liposomes having a uniform particle size and entrapping the polymerizable monomer therein can be recovered. Removing the polymerizable monomer not entrapped in the liposomes from the external solution by dialysis, the monomer entrapped in liposomes should be polymerized with a polymerization initiator.

Then, a group reactive with the crosslinking agent should be introduced into the molecule of an antibody or antigen. Mixing and reacting then the thus prepared liposomes with the modified antibody or the modified antigen in a buffer solution, the surface of the liposomes is immobilized with the antibody or the antigen.

As the antibody immobilized onto the liposome surface in accordance with the present invention, use may be made of a monoclonal antibody and/or a polyclonal antibody recognizing an antigenic substance to be assayed. The immunoagglutination reagent comprising a combination of such antibody and such liposomes are grouped into the following types; an immunoagglutination reagent immobilizing only a monoclonal antibody onto the liposome surface; an immunoagglutination reagent immobilizing only a polyclonal antibody onto the liposome surface; an immunoagglutination reagent immobilizing a monoclonal antibody and a polyclonal antibody onto the same liposome surface; and an immunoagglutination reagent comprising a mixture of a monoclonal antibody immobilized onto the surface of liposome and a polyclonal antibody immobilized onto the surface of another liposome.

In the immunoagglutination reagent comprising a combination of a monoclonal antibody and a polyclonal antibody as described above, a monoclonal antibody with higher affinity with an antigen complements the lower affinity of a polyclonal antibody whereas a polyclonal antibody with higher agglutination potency can avoid the laborious work in selecting a combination of monoclonal antibodies.

As the antibody immobilized onto the liposome surface, use may be made of the IgG fraction, or otherwise, use may be made of one of the fragment, i.e., F(ab')₂ fragment. In accordance with the present invention, the antigen to be immobilized onto the liposome surface includes plasma proteins and virus proteins and the like.

The antigenic substance to be assayed by the immunoanalytical method with the immunoagglutination reagent in accordance with the present invention includes hormones (for example, insulin, HCG-β, growth hormone, TSH, LH, FSH, prolactin, thyroxine, triiodothyronine, gastrin, glucagon, somatostatin, etc.); enzymes (for example, elastase, amylase, protease, lipase, ribonuclease, enolase, alkaliphosphatase, etc. ); serum proteins (for example, IgG, IgA, IgM, IgE, IgD, RF, SLO, macroglobulin, TBG, sugar proteins, lipopolysaccharides, apo-AI, AII, B, CI, CII, CIII, Proteins D, E, F, etc.); tumor-related antigens (for example, CEA, α-fetoprotein, ferritin, POA, CA19-9, CA125, etc.); DNA binding proteinous factors; cytokines (for example, interferon, interleukin-1, interleukin-2, etc.); a variety of bacteria, viruses, protozoa (for example, fungus, streptococcus, hepatitis virus, herpesvirus, HIV virus, Toxoplasma gondii, Plasmodium, Entamoeba histolytica, etc.); and the like. Among them, application is more suitable of serum proteins, turmor-related antigens and viruses, which particularly need to assay a great number of samples at a time for a short period.

The antibody to be assayed includes for example antibodies against viruses (for example, HIV, ATL, HB, etc.), antinuclear factor and the like.

By the immunoanalytical method using the immunoagglutination reagent in accordance with the present invention, a sample containing a subjective substance should be assayed for example as follows. Firstly, mixing the immunoagglutination reagent with a sample containing a subjective substance in an appropriate buffer (for example, TES buffer), thereby inducing an antigen-antibody reaction, liposomes are agglutinated. The decrease in photo-transmission depending on the level of the agglutination should be analyzed with a spectrophotometer or an automatic analyzer (HITACHI TYPE 705, 7050, 7150, 736, 7070, etc.). Then, comparing the decrease with a standard curve preliminary prepared, the subjective substance can be assayed.

### BRIEF DESCRIPTION OF THE DRAWINGS

Fig. 1 shows a standard curve of an immunoagglutination reagent for CRP assay, comprising as the carriers liposomes entrapping a gelled compound and liposomes entrapping TES buffer; and
Fig. 2 shows a standard curve of an immunoagglutination reagent for RF assay, comprising as the carriers liposomes entrapping a gelled compound and liposomes entrapping TES buffer.

### BEST EMBODIMENTS FOR CARRYING OUT THE INVENTION

In the following Example 1, an immunoagglutination reagent for CRP antigen assay by liposome turbidimetry will be produced in accordance with the present invention. In the Example 1, use is made of the following reagents and assaying samples.

Unless otherwise stated, the same reagents and samples are used in Example 2 and Comparative Examples 1 and 2 described below.
DPPC: dipalmitoylphosphatidylcholine
Chol: cholesterol
DTP-DPPE: dithiopyridylated dipalmitoylphosphatidyl ethanolamine
Aam solution: prepared by dissolving acrylamide and Bisacrylamide in distilled water to a final gel concentration of 2.5 % and a final crosslinking degree of 5 %.

TES buffer solution: prepared by dissolving TES, sodium chloride and Miracle Mornone (as Trade Name; manufactured by Katayama Kagaku Industry Research Institute ) in distilled water to final concentrations of 10 mM, 150 mM and 0.01 %, respectively, and then adjusting the mixture to pH 7.5 via sodium hydroxide.

Acetate buffer: prepared by dissolving acetic acid, sodium chloride, Miracle Mornone (as Trade Name; manufactured by Katayama Kagaku Industry Research Institute) in distilled water to final concentrations of 100 mM, 150 mM and 0.01 %, respectively, and then adjusting the mixture to pH 4.5 via sodium hydroxide.
SPDP: N-hydroxysuccinimidyl 3-(2-pyridyldithio) propionate
DTT: dithiothreitol
Goat anti-CRP antibody: dissolved in 10 mM Hepes buffer (containing 150 mM sodium chloride, pH 7.5 ).

### [Example 1]

### (A) Preparation of liposomes

Charging a chloroform solution containing DPPC (50 µmol), Chol (50 µmol), and DTP-DPPE (2.5 µmol) in a 100-ml pear-type flask, and thereafter evaporating the solution under warming, chloroform is distilled off. The lipid together with the cholesterol forms a thin film on the inner wall surface of the flask. Placing the flask under reduced pressure for more than one hour, the solvent is completely distilled off.

Adding then the Aam solution (2 ml) and glass beads (2 g), followed by vigorous agitation, the thin film is peeled off. After removing the glass beads and sizing the lipid suspension to 0.2 µm by using an extruder, liposomes of a uniform. particle size are prepared. The Aam solution not entrapped in the liposomes is removed by 24-hour dialysis in TES.

Adding subsequently ammonium persulfate (33 µl) and N, N, N', N'-tetramethylethylene diamine (14 µl) dissolved in distilled water to final individual concentrations both at 10%, for reaction at room temperature for 4 to 16 hours, the entrapped Aam solution is polymerized. Removing the ammonium persulfate and N, N, N', N'-tetramethylethylene diamine after such reaction by 24-hour dialysis in TES, liposomes entrapping the gelled compound are prepared. The concentration of the thus produced liposomes is determined by phosphorous assay.

### (B) Introduction of a reactive group into an antibody

30 mM SPDP (10 µl; dissolved in ethanol ) is added to and reacted with 10 mg/ml goat anti-CRP antibody (1 ml) at room temperature for 30 minutes. The resulting mixture is subjected to gel filtration on Sephadex G-25 equilibrated with an acetate buffer, the unreactive SPDP is removed at the time of exchange of the buffer solution. DTT is added to the resulting antibody solution to a final concentration of 10 mM for reaction at room temperature for 30 minutes. The resulting solution is then subjected to gel filtration on Sephadex G-25 equilibrated with TES. Then, DTT is removed at the time of exchange of the buffer solution. The absorbance of the resulting antibody solution is measured at 280 nm with a spectrophotometer to determine the concentration.

### (C) Preparation of antibody-bearing liposomes

The liposomes prepared in the item (A) (1 ml; 10 nm phosphorous concentration) are mixed and reacted with the 5 mg/ml antibody solution prepared in the item (B) (1 ml), at 4 ° C for 16 to 20 hours. Then, the resulting mixture is subjected to gel filtration on Sepharose CL-4B equilibrated with TES, to remove the unreactive antibody, thus fractionating the liposome fraction. The phosphorous assay is done of the liposomes covalently coated with the resulting antibody, to determine the amount of antibody on liposome and to subsequently determine the amount of protein per phosphorous by the Lawry method. The particle size is determined with an analyzer of particle size distribution.

### (D) Assay of CRP antigen

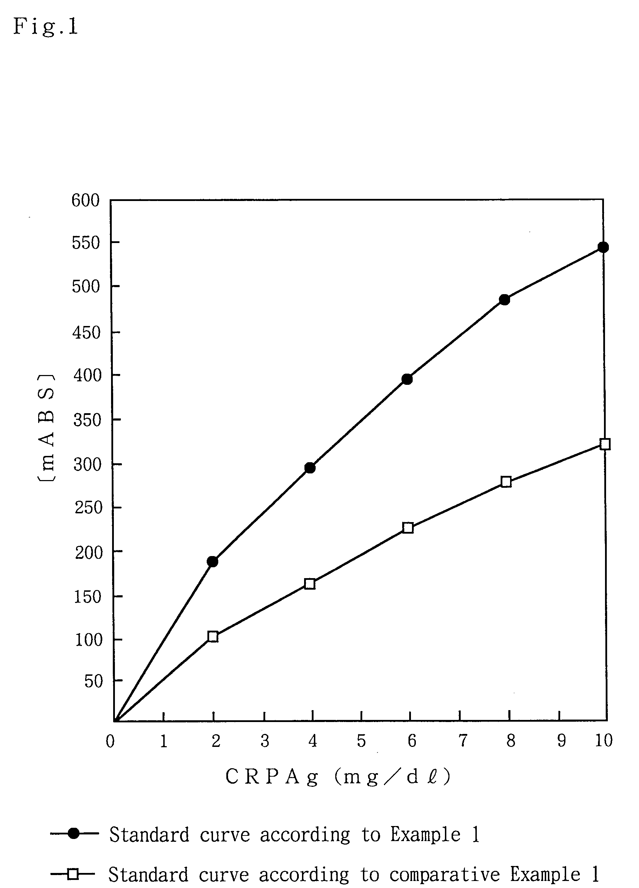

The liposomes covalently coated with the antibody prepared in the item (C) are diluted with TES to a final phosphorous concentration of 1 mM, and the resulting solution is designated the immunoagglutination reagent. Using a HITACHI TYPE 7150 automatic system as the analyzer, and using purified CRP antigen (7 µl) and the immunoagglutination reagent(280 µl), the CRP antigen is assayed under the following assay conditions; a principal wave length of 340 nm and a secondary wave length of 700 nm at an analytical point of 24 to 50. The results are shown in Fig. 1.

In Fig. 1, the longitudinal axis represents the change in absorbance of each sample in unit mABS. The absorbance shown in the figure is calculated by subtracting the blank of the reagent at zero concentration of the CRP antigen from the original absorbance. The transverse axis represents the concentration of the CRP antigen in unit mg/dl.

### [Comparative Example 1]

The same procedure as in Example 1 is carried out, except for the use of TES instead of the Aam solution as in the item (A) with no use of ammonium persulfate and N, N, N', N'-tetramethylethylene diamine. The results are shown in Fig. 1. The longitudinal and transverse axes independently represent the same as described above. As apparently shown in Fig. 1, it is indicated that a highly sensitive immunoagglutination reagent for CRP antigen assay can be produced, from the fact that by entrapping the gelled compound into liposomes thereby increasing the refractive index, the change in absorbance is enhanced by about 1.7 fold after the reaction of the antibody-bearing liposomes.

The liposome used in the Example 1 and Comparative Example 1 is sensitized at an amount of about 150 g per 1 mole phosphorous.

### [Example 2]

### Immune agglutination reagent for RF assay by liposome turbidimetry

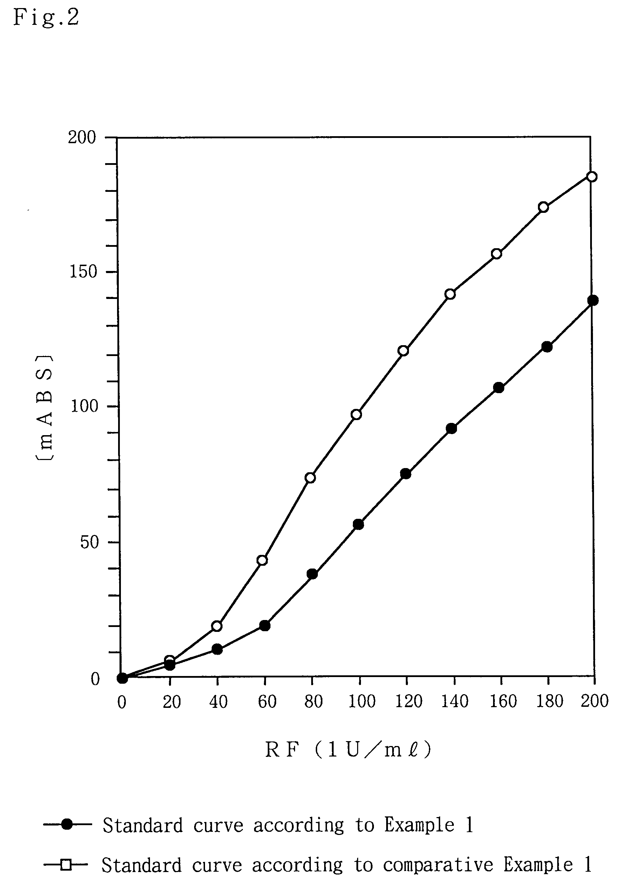

Liposomes are prepared as described above in Example 1. Also, antigen-bearing liposomes are prepared in the same manner as in Example 1, except for the use of a heat-aggregated human IgG as an antigen instead of the goat anti-CRP antibody. RF assay:
The antigen-sensitized liposomes prepared above are diluted with TES to a final phosphorous concentration of 0.25 mM, and the resulting solution is designated a second reagent. Herein, a first reagent is prepared by using TES. Using a HITACHI TYPE 7150 automatic system as the analyzer, and using RF-positive serum (10 µl) and the first reagent (300 µl) and the second reagent (100 µl), RF is determined under the following conditions; a principal wave length of 340 nm and a secondary wave length of 700 nm at an analytical point of 24 to 50. The results are shown in Fig. 2.

In Fig. 2, the longitudinal axis represents the change in absorbance of each sample in unit mABS. The absorbance shown is calculated by subtracting the blank of the reagent at zero concentration of the RF antigen from the original absorbance. The transverse axis represents the concentration of the RF antigen.

### [Comparative Example 2]

Sensitizing the heat-aggregated human IgG in the same manner as in Comparative Example 1, RF is assayed in the same manner as in Example 2 (A). The results are shown in Fig. 2. As apparently shown in Fig. 2, it is indicated that a highly sensitive RF diagnostic assay reagent can be produced, from the fact that by entrapping the gelled compound into liposomes as in the same manner as in Example 1 thereby increasing the refractive index, the absorbance change after the liposome reaction is increased by about 1.4 fold. In the Comparative Example 2 as in Example 2, the sensitized amount is about 210 g per 1 mole phosphorous.

Figs. 1 and 2 indicate the following. It is confirmed that by enveloping a water-soluble polymer compound or a gelled compound into liposome as the carrier of an imm unoagglutinationan reagent (with the fundamental assay principal) based on agglutination reaction, the assay sensitivity can be increased.

### Industrial Applicability

Assaying an antigen or an antibody contained in a sample using the immunoagglutination reagent in accordance with the present invention, the change in turbidity due to the liposome agglutination via the antigen-antibody reaction can be enhanced, whereby a lower concentration of a substance in the sample can be assayed at a high sensitivity.

In accordance with the present invention, furthermore, liposomes with less antigenicity is used as the carrier. Thus, non-specific agglutination hardly occurs.

Because assaying can be done with an automatic system by means of the immunoagglutination reagent in accordance with the present invention, a great number of samples can be assayed at a time for a short period. Assaying can be done in a stable manner because no contamination of such automatic system occurs.

## Claims

1. An immunoagglutination reagent to assay an agglutination level caused by an antigen-antibody reaction, comprising:
(1) liposomes being used as the carrier of the reagent for immunoagglutination,
(2) an antibody or an antigen being immobilized onto the surface of the liposome, and
(3) the liposomes entrapping a water-soluble polymer compound or a gelled compound.

2. An immunoagglutination reagent according to claim 1, wherein the antibody or the antigen is covalently immobilized onto the surface of the liposome.

3. An immunoanalytical method comprising reacting the immunoagglutination reagent according to claim 1 or 2 with a sample to be assayed, and measuring the level of the liposome agglutination caused by the antigen-antibody reaction.
